# EUROPÄISCHE PATENTSCHRIFT

(11) **EP 3 064 166 B1**
(45) Veröffentlichungstag und Bekanntmachung des Hinweises auf die Patenterteilung: **04.07.2018**
(21) Anmeldenummer: 16157312.6
(22) Anmeldetag: 25.02.2016
(51) Int. Cl.: A61C 3/02, F21V 31/00, A61B 1/06, H01L 25/16, A61C 1/08, F21V 33/00, F21V 3/00, F21K 9/90, F21V 17/10, F21W 131/20, F21Y 101/00, F21K 9/20

(54) **HERMETISCH ABGEDICHTETE LED-LEUCHTE SOWIE VERFAHREN ZUR HERSTELLUNG EINER HERMETISCH ABGEDICHTETEN LED-LEUCHTE**
HERMETICALLY SEALED LED LIGHT, AND METHOD FOR MANUFACTURING A HERMETICALLY SEALED LED LIGHT
ÉCLAIRAGE DEL ETANCHEIFIE ET PROCEDE DE FABRICATION D'UN ECLAIRAGE DEL ETANCHEIFIE

(30) Priorität: 06.03.2015 DE 102015103331; 10.03.2015 DE 102015103507
(43) Veröffentlichungstag der Anmeldung: 07.09.2016
(73) Patentinhaber: Schott AG, 55122 Mainz (DE)
(72) Erfinder: RAKOBRANDT, Christian, 84034 Landshut (DE); GINDELE, Frank, 85301 Schweitenkirchen (DE)
(74) Vertreter: Blumbach · Zinngrebe Patent- und Rechtsanwälte

(56) Entgegenhaltungen:
- EP-A1- 2 548 530
- EP-A1- 2 842 513
- Schott Ag: "Die erste ringförmige autoklavierbare High Brightness LED von SCHOTT", , 9. Februar 2015 (2015-02-09), XP055290210, www.schott.com Gefunden im Internet: URL:http://www.schott.com/newsfiles/com/01 3_2015_schott-ring-leds-for-dental-and-med ical-applications_de_final.pdf [gefunden am 2016-07-21]

## Beschreibung

### Gebiet der Erfindung

Die Erfindung betrifft eine hermetisch abgedichtete LED-Leuchte sowie ein Verfahren zur Herstellung einer hermetisch abgedichteten LED-Leuchte. Insbesondere betrifft die Erfindung eine LED-Leuchte, welche im medizinischen Bereich, also für ärztliche Geräte und Instrumente, verwendet wird.

### Hintergrund der Erfindung

Im ärztlichen Bereich, insbesondere bei Geräten von Zahnärzten, finden vermehrt LED-Leuchten Verwendung, welche insbesondere auch Teil von ärztlichen Instrumenten sein können. Diese dienen Beleuchtungszwecken, beispielsweise des Ausleuchtens der Mundhöhle eines Patienten, dem Aufspüren von Defektstellen, etwa von Karies, wenn beispielsweise Licht einer bestimmten Wellenlänge, welches das Auffinden von Defektstellen erleichtert, emittiert wird, als Beleuchtung einer Kamera, insbesondere der eines Endoskops, sowie dem Aushärten von Füllmaterialen, etwa beim Aushärten von mittels UV-Licht aushärtbaren Kunststoffen.

Typischerweise unterliegen derartige Leuchten hohen Belastungen, insbesondere müssen die meisten medizinischen Geräte hermetisch abgedichtet und in manchen Fällen sogar autoklavierbar sein.

Die verwendeten Vorrichtungen sollten zudem langlebig sein und keine schädlichen Stoffe abgeben.

Insbesondere bei zahnärztlichen Instrumenten sind Griffstücke bekannt, welche eine LED-Lichtquelle aufweisen. Die LED-Lichtquelle ist dabei zumeist in das Gehäuse des Griffstücks integriert und das von der LED-Lichtquelle abgestrahlte Licht wird mittels eines Lichtleiters zu einem Fenster im vorderen Bereich des Griffstücks geleitet, von wo aus dieses in den Mundraum abgestrahlt wird.

Bekannte LED-Leuchten, welche beispielsweise mit einer polymeren Vergussmasse abgedichtet sind, sind in der Regel für einen Einsatz zumindest an der Außenseite eines derartigen ärztlichen Gerätes nicht hinreichend geeignet.

Das Dokument Schott: "Die erste ringförmige autoklavierbare High Brightness LED von Schott", 09. Februar 2015, XP055290210, www.schott.com, zeigt eine autoklavierbare Ring-LED.

Das Dokument EP 2 548 530 A1 zeigt eine Ring-LED für ein ärztliches Instrument, welche Fluidkanäle umfasst.

Das Dokument EP 2 842 513 A1 zeigt eine verkapselte Leuchte für ein dentales Instrument, bei welcher ein Trägerelement mit einer Metallhülse verlötet ist.

### Aufgabe der Erfindung

Der Erfindung liegt demgegenüber die Aufgabe zugrunde, eine hermetisch abgedichtete LED-Leuchte bereitzustellen, welche gut in ein ärztliches Instrument integrierbar und den erhöhten Anforderungen im medizinischen Bereich, was Haltbarkeit und Dichtigkeit angeht, gewachsen ist.

### Zusammenfassung der Erfindung

Die Aufgabe der Erfindung wird bereits durch eine hermetisch abgedichtete LED-Leuchte sowie ein Verfahren zum Herstellen einer hermetisch abgedichteten LED-Leuchte nach einem der unabhängigen Ansprüche gelöst.

Bevorzugte Ausführungsformen und Weiterbildungen der Erfindung sind dem Gegenstand der jeweiligen Unteransprüche zu entnehmen.

Die Erfindung betrifft eine hermetisch abgedichtete LED-Leuchte, also eine LED-Leuchte mit einem Gehäuse, welches zumindest flüssigkeitsdicht ist.

Die LED-Leuchte kann einen Sockel mit einer Mehrzahl von LEDs umfassen.

Vorzugsweise handelt es sich bei den LEDs um als Chip ausgebildete LEDs, welche direkt auf den Sockel gebondet sind.

Derartige LED-Chips können LEDs mit hoher Leistung aufweisen, die Wärme lässt sich gut über den Sockel abführen und die Bauteile sind kompakt ausgebildet.

Der Sockel ist aus einer Keramik ausgebildet. Insbesondere ist der Sockel aus einem Aluminiumoxid und/oder einem Aluminiumnitrid ausgebildet.

Weiter weist die LED-Leuchte eine Metallkappe mit zumindest einem Fenster auf. Vorzugsweise weist die Metallkappe mehrere Fenster auf, welche jeweils den LEDs gegenüberliegen und durch die das Licht aus der LED-Leuchte austritt.

Die Metallkappe ist auf den Sockel gelötet und die LED-Leuchte kann des Weiteren einen Kanal zum Einbringen eines elektrischen, optischen und/oder mechanischen Bauelements aufweisen, welcher sich sowohl durch Sockel als auch durch Kappe erstreckt.

Durch die Kombination eines Sockels aus Keramik mit einer erfindungsgemäß mittels eines Gold-Zinn-Lots aufgelöteten Metallkappe konnte ein Bauelement in Form einer LED-Leuchte bereitgestellt werden, welches trotz eines Kanals, der die Bauteile durchdringt, eine hohe Dichtigkeit aufweist.

Der Kanal dient der Aufnahme eines elektrischen oder mechanischen Bauelements, welches insbesondere Teil eines verwendeten ärztlichen Gerätes ist.

Beispielsweise kann in dem Kanal ein Bildsensor, insbesondere eine Kamera angeordnet sein, für welche die LED-Leuchte als Lichtquelle dient.

Der Kanal kann dabei eine beliebige Form haben, insbesondere eine konische, kreiszylindrische oder auch eckige Form. Weiter ist denkbar, den Kanal gestuft auszubilden, beispielsweise indem Sockel und Kappe einen Kanal mit unterschiedlichem Durchmesser aufweisen. Diese Stufe kann sodann als Anschlag zum verbesserten Halten eines mechanischen oder elektrischen Bauelements verwendet werden.

Bei einer bevorzugten Ausführungsform ist die LED-Leuchte ringförmig mit im Wesentlichen mittig angeordnetem Kanal ausgebildet.

Die LEDs sind vorzugsweise über den Umfang des so gebildeten Rings verteilt. Die LED-Leuchte kann insbesondere 4 bis 10 LEDs aufweisen.

Eine derartige Leuchte kann insbesondere auch am Kopfstück eines ärztlichen Instruments, beispielsweise auch eines Zahnarztbohrers angeordnet sein. Dabei kann die Antriebswelle des Bohrers und/oder der Bohrer selbst durch den Kanal ragen und die LEDs dienen insbesondere zur Ausleuchtung.

Vorzugsweise umfasst die LED-Leuchte eine Mehrzahl von Fenstern, welche ringförmig verteilt sind.

Bei einer Weiterbildung der Erfindung sind die Fenster als Linsen ausgebildet.

Insbesondere kann über die Fenster das Licht der LEDs fokussiert werden.

Bei einer bevorzugten Ausführungsform sind die Fenster jeweils in einen Kanal der Metallkappe eingelassen. Die Kanäle, in die die Fenster eingelassen sind, haben vorzugsweise eine Höhe zwischen 0,15 und 20 mm. Vorzugsweise reichen die Kanäle jeweils bis zum Sockel, d.h. unmittelbar angrenzend zum jeweiligen Kanal sind Metallkappe und Sockel verlötet.

Es ist insbesondere vorgesehen, dass die Metallkappe vorzugsweise zylindrisch, insbesondere kreiszylindrisch ausgebildete Kanäle aufweist, in welche Glasfenster, insbesondere in Form von Linsen, eingeschmolzen sind.

Für die Herstellung einer derartigen Metallkappe kann jeweils ein Stück eines Glasstabs in jeweils einen Kanal eingelegt werden. Durch ein Erwärmen schmilzt das Glas und es bildet sich aufgrund der Oberflächenspannung des Glases eine Linse aus.

Bei einer Ausführungsvariante umfasst die LED-Leuchte eine Mehrzahl von Fenstern, wobei die Metallkappe angrenzend zu den Fenstern eine Einsenkung umfasst.

Insbesondere umfasst die Metallkappe an der Oberseite eine Einsenkung, an welche ein Kanal angrenzt.

Wird nunmehr ein Glasfenster in den Kanal eingeschmolzen, wobei sich eine Linse ausbildet, steht die Linse aufgrund der Einsenkung nicht über die Oberseite der Metallkappe hinaus, so dass, wenn die LED-Leuchte ohne weitere Schutzscheibe verwendet wird, die Fenster bei Berührung eines Gegenstandes nicht beschädigt werden, da die Metallkappe anstößt.

Bei einer Ausführungsform der Erfindung ist in den Kanal unterhalb des Fensters ein Konverter eingelassen. Insbesondere kann ein Konverter mit einer Trägermatrix aus Silikon verwendet werden, in welchem fluoreszierende Partikel, beispielsweise Phosphor, YAG etc., eingebettet sind. Der Konverter lässt sich in den Kanal besonders einfach einbringen, etwa durch Einkleben. Die Verwendung eines Konverters mit einer Silikonmatrix resultiert in einer einfachen Herstellung und einer hohen Temperaturstabilität. Weiter ist auch die Verwendung von Konvertern mit anorganischer Matrix denkbar.

Durch das Einschmelzen eines Glasfensters in einem Kanal, welches vorzugsweise möglichst dicht vor einem optional verwendeten Konverter angeordnet ist, kann eine LED-Leuchte mit großem Abstrahlwinkel bereitgestellt werden.

Insbesondere kann der Abstrahlwinkel mehr als 85°, vorzugsweise mehr als 90° und besonders bevorzugt mehr als 94° betragen. Unter dem Abstrahlwinkel im Sinne der Erfindung wird der von seitlichen Punkten mit halber maximaler Lichtstärke eingeschlossene Winkel verstanden.

Die LED-Leuchte kann getrennt ansteuerbare LEDs verschiedener Lichtfarbe umfassen, wobei der Abstrahlwinkel des Lichts von zumindest zwei getrennt ansteuerbaren LEDs unterschiedlich ist.

Die LED-Leuchte umfasst für verschiedene Verwendungen also LEDs in unterschiedlicher Lichtfarbe. Im Falle der Bereitstellung einer LED-Leuchte für den zahnärztlichen Bereich können dies beispielsweise Leuchten mit im Wesentlichen weißem Licht zum Ausleuchten der Mundhöhle, LEDs spezieller Lichtfarbe zum Erkennen von Karies oder Zahnbelegen sowie LEDs, welche UV-Licht emittieren, sein, welche dem Aushärten von Kunststoff dienen. Diese sind getrennt ansteuerbar. Hierzu umfasst der Sockel eine Mehrzahl elektrischer Durchführungen für die jeweiligen LEDs oder LED-Gruppen.

Es ist insbesondere vorgesehen, dass zumindest eine LED einen Abstrahlwinkel von weniger als 60° und eine andere LED einen Abstrahlwinkel von mehr als 70° aufweist. Weiter ist insbesondere vorgesehen, dass sich der Abstrahlwinkel von mindestens zwei LEDs um zumindest 10°, vorzugsweise um zumindest 20° unterscheidet.

Hierzu werden vorzugsweise Fenster verwendet, welche als Linsen mit unterschiedlicher Brennweite und/oder mit unterschiedlichem Öffnungswinkel ausgebildet sind.

Im Falle des Einschmelzens von Glas können diese beispielsweise durch eine unterschiedliche Form des Kanals und/oder durch ein unterschiedliches Glas bereitgestellt werden.

Beispielsweise für LEDs, die UV-Licht emittieren, kann eine Fokussierung des Lichtes und das Abstrahlen mit geringem Abstrahlwinkel sinnvoll sein, wohingegen LEDs zum Ausleuchten des Mundraums einen breiteren Abstrahlwinkel aufweisen.

Die LED-Leuchte ist insbesondere Teil eines ärztlichen Instruments, wobei die LED-Leuchte derart in das ärztliche Instrument eingelassen ist, dass die LED-Leuchte direkt nach außen abstrahlt. Hierunter wird verstanden, dass das Licht nicht über einen Lichtleiter zu einem Austrittsfenster geleitet wird. Es ist aber denkbar, dass in Emissionsrichtung noch ein weiteres Fenster oder eine Schutzscheibe vorgesehen ist. Vorzugsweise bildet die Metallkappe im Bereich der Leuchte samt ihren Fenstern aber den Abschluss des ärztlichen Instruments.

Die Erfindung betrifft des Weiteren ein Verfahren zum Herstellen einer LED-Leuchte, insbesondere ein Verfahren zum Herstellen einer LED-Leuchte, wie sie vorstehend beschrieben wurde.

Dabei wird zunächst ein Sockel aus Keramik mit zumindest einer LED bestückt, insbesondere mit einer LED, welche auf einem Chip angeordnet ist. Sodann wird mittels eines Metall-Lots eine Metallkappe auf den Sockel gelötet.

Vorzugsweise wird bereits vor dem Verlöten die Metallkappe mit zumindest einem Fenster versehen, insbesondere durch Aufschmelzen eines Glases in jeweils einem Kanal der Metallkappe.

Denkbar ist auch, das zumindest eine Fenster als Druckeinglasung auszugestalten. Die aus Glas hergestellten Fenster sind vorzugsweise aus einem Material mit guter chemischer Beständigkeit, insbesondere aus einem Silikatglas, beispielsweise aus einem Borosilikatglas, ausgebildet.

Es sind sodann nur noch die Elemente Metallkappe nebst Fenster und Sockel aus Keramik, zusammenzufügen.

Es hat sich gezeigt, dass durch die erfindungsgemäße Verwendung eines Gold-Zinn-Lots eine stabile und hermetisch dichte Verbindung erzielt werden kann, ohne dass die LED-Leuchte derart heiß wird, dass die LED, insbesondere die als Chip ausgebildete LED, beschädigt wird.

Durch die Verwendung eines Gold-Zinn-Lots kann ein autoklavierbares Gehäuse bereit gestellt werden.

Weiter ist diese Verbindung auch bei eventuell folgenden Lötprozessen, insbesondere einem Reflowprozess oder bei der Verwendung eines Zinn-Silber-Kupfer-Lots beständig.

Vorzugsweise wird ein Lot, insbesondere ein Gold-Zinn-Lot verwendet, dessen Schmelztemperatur bei unter 300°C, besonders bevorzugt bei unter 280°C, liegt.

Weiter weist vorzugsweise der Sockel aus Keramik elektrische Durchführungen auf, welche durch einen Metall-Lot hermetisch verriegelt sind.

Auch diese Durchführungen, über die die LEDs kontaktiert sind, werden vorzugsweise vor dem Verlöten von Sockel und Metallkappe ausgebildet.

Bei einer Weiterbildung der Erfindung wird zumindest die Metallkappe mit einer Beschichtung versehen, insbesondere mit einer nickel- und/oder goldhaltigen Beschichtung. Insbesondere wird eine Beschichtung aus Hartgold aufgebracht.

Die Metallkappe besteht vorzugsweise aus einem Edelstahl, insbesondere aus einem Edelstahl mit einem geringen Wärmeausdehnungskoeffizienten. So ist die Metallkappe an den niedrigen Wärmeausdehnungskoeffizienten der Keramik angepasst. Insbesondere hat das Material der Metallkappe einen thermischen Wärmeausdehnungskoeffizienten α bei Raumtemperatur von weniger als 15, bevorzugt weniger als 8 und besonders bevorzugt weniger als 5 ppm/K.

Insbesondere besteht die Metallkappe aus einem ferritischen Edelstahl.

Bei einer Ausführungsform der Erfindung weichen die thermischen Ausdehnungskoeffizienten α von Metallkappe und dem Material der Fenster weniger als 5 ppm/K, vorzugsweise weniger als 1 ppm/K voneinander ab. Gleiches gilt vorzugsweise für die thermischem Ausdehnungskoeffizienten von Sockel und Metallkappe.

Als Lot für die Durchführungen der Kontaktierung der LEDs kann beispielsweise ein Gold-Zinn-, ein Zinn-, ein Kupfer- oder Silber-Lot verwendet werden.

Durch die Erfindung konnte eine hermetisch abgedichtete LED-Leuchte bereitgestellt werden, welche sogar autoklavierbar ist.

### Kurzbeschreibung der Zeichnungen

Der Gegenstand der Erfindung soll im Folgenden bezugnehmend auf Ausführungsbeispiele näher erläutert werden.
Fig. 1 zeigt eine Draufsicht auf ein Ausführungsbeispiel einer hermetisch abgedichteten LED-Leuchte.
Fig. 2 zeigt eine Schnittansicht der Fig. 1.
Fig. 3 zeigt eine Detaildarstellung der Fig. 2.
Fig. 4 zeigt eine Draufsicht auf die Unterseite.
Fig. 5 zeigt in einer schematischen Darstellung die Verwendung der erfindungsgemäßen hermetisch abgedichteten LED-Leuchte in Verbindung mit einem Drittsensor.
Fig. 6 zeigt eine schematische Ansicht eines ärztlichen Instrumentes, in welches eine erfindungsgemäße, hermetisch abgedichtete LED-Leuchte eingebaut ist.
Fig. 7 zeigt ein anderes Ausführungsbeispiel einer hermetisch abgedichteten LED-Leuchte, welche nicht ringförmig ausgebildet ist.

### Detaillierte Beschreibung der Zeichnungen

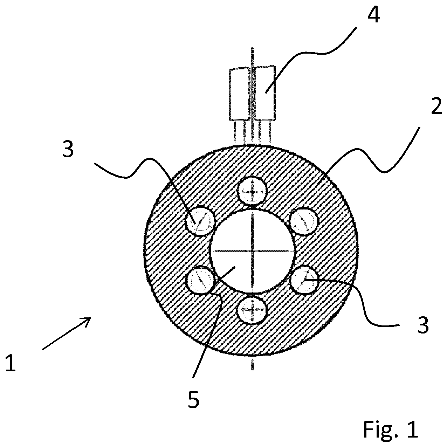

Fig. 1 zeigt in einer Draufsicht ein Ausführungsbeispiel einer erfindungsgemäßen, hermetisch abgedichteten LED-Leuchte 1.

Die LED-Leuchte 1 ist in diesem Ausführungsbeispiel ringförmig ausgebildet. Zu erkennen ist auf der Oberseite die Metallkappe 2, welche eine Mehrzahl von Fenstern 3 aufweist, durch die das Licht der darunterliegenden LEDs abgestrahlt wird.

Über die Zuleitung 4 kann die LED-Leuchte 1 angeschlossen werden.

Die in diesem Ausführungsbeispiel dargestellten sechs Fenster 3 sind über den Umfang der LED-Leuchte 1 verteilt.

Weiter zu erkennen ist ein mittig angeordneter Kanal 5 mit kreisförmigem Querschnitt, welcher der Aufnahme eines elektrischen, optischen oder mechanischen Bauelements dient.

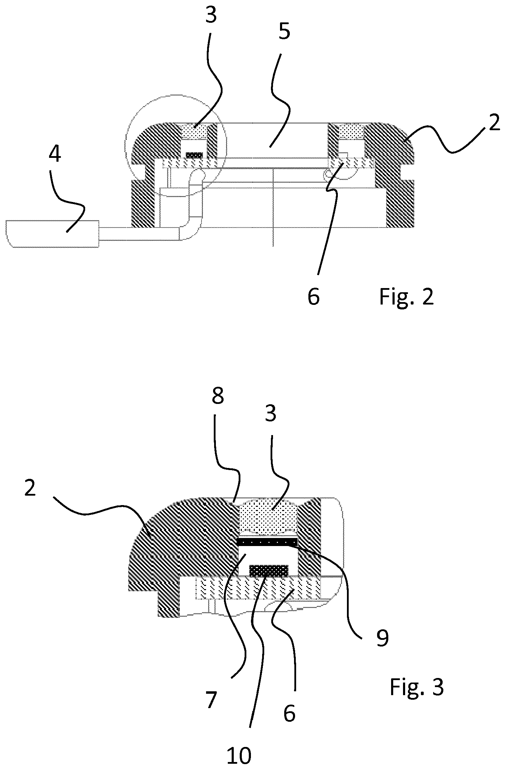

Fig. 2 zeigt eine Schnittansicht der in Fig. 1 dargestellten LED-Leuchte.

Zu erkennen ist, dass die Metallkappe 2, welche aus einem Edelstahl besteht, topfförmig ausgebildet ist.

Ein Sockel 6 aus einer Keramik, insbesondere aus Aluminiumoxid, ist von der Unterseite in die topfförmige Metallkappe 2 eingelassen und mittels eines Gold-Zinn-Lots mit der Metallkappe verlötet.

Der plattenförmige Sockel 6 ist ebenfalls ringförmig ausgebildet, so dass der Kanal 5 sowohl durch den Sockel 6 als auch durch die Metallkappe 2 verläuft.

Der Sockel 6 besitzt Durchführungen (nicht dargestellt), über welche über die Zuleitung 4 Strom zur Ansteuerung der LEDs auf die Oberseite geleitet wird.

Fig. 3 zeigt eine Detaildarstellung der Fig. 2, und zwar den Bereich, in welchem ein Fenster 3 angeordnet ist.

Zu erkennen ist, dass der Sockel 6 des Fensters 3 mit einer LED 10 bestückt ist. Die LED 10 ist als Chip ausgebildet. Es handelt sich insbesondere um ein SMD- (Surface Mounted Device) Bauelement.

Auf der Oberseite des Sockels 6 können sich Zuleitungen, etwa in Form von Flachleiterbahnen, zur Kontaktierung der LED 10 befinden (nicht dargestellt).

Zu erkennen ist ferner, dass das Fenster 3 linsenförmig ausgebildet ist.

Das Fenster 3 ist in einen Kanal 7 eingelassen, welcher seinerseits in die Metallkappe 2 eingebracht ist.

Die linsenförmige Ausgestaltung des Fensters 3 kommt durch die Oberflächenspannung des Glases zustande, indem dieses hergestellt wird, indem ein Glasstab in den Kanal 7 eingelegt und sodann aufgeschmolzen wird.

Oberhalb des Kanals 7 befindet sich eine randseitige Einsenkung 8.

Aufgrund der Einsenkung 8 ragt das linsenförmig ausgestaltete Fenster 3 nicht hervor.

Optional kann, je nach gewünschter Lichtfarbe, in den Kanal 7 ein Konverter 9 eingebracht sein. Es handelt sich insbesondere um einen Konverter mit einer Silikonmatrix, welcher in den Kanal 7 eingeklebt wird.

Zu erkennen ist ferner, dass der Sockel 6 mit seiner Oberseite auf die Unterseite der topfförmigen Metallkappe 2 gelötet ist.

Randseitig ist in diesem Ausführungsbeispiel der Sockel 6 von der Metallkappe 2 beabstandet.

Durch das Verlöten von Sockel 6 und Metallkappe 2 sowie durch das eingeschmolzene Fenster 3 wird eine hermetische Abdichtung der LED 10 bereitgestellt.

Kontaktführungen im Sockel 6 sind vorzugsweise ebenfalls mit einem Lot aufgefüllt.

Für eine Montage der erfindungsgemäßen LED-Leuchte wird zunächst die Metallkappe mit den eingeschmolzenen Fenstern hergestellt und separat hiervon wird der Sockel 6 mit den LEDs 10 bestückt.

Diese beiden Hauptkomponenten werden sodann mittels eines Gold-Zinn-Lots mit einer Schmelztemperatur von unter 340 °C, bevorzugt unter 325 °C, besonders bevorzugt unter 300 °C miteinander verlötet.

Das erfindungsgemäße Verfahren eignet sich nicht nur für die hier dargestellte ringförmige LED-Leuchte, sondern auch für andere Arten von Leuchten, also insbesondere auch solche ohne einen mittigen Kanal.

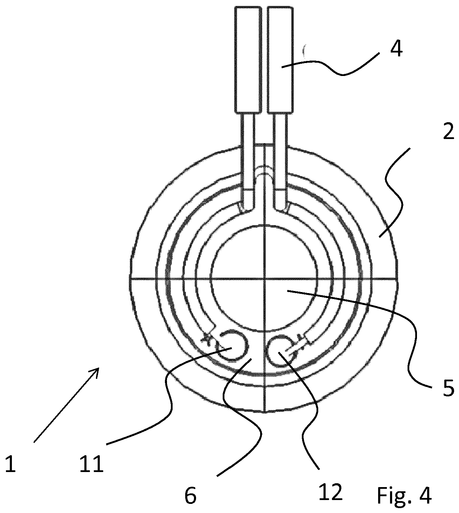

Fig. 4 zeigt eine Draufsicht von der Unterseite der LED-Leuchte 1.

Zu erkennen ist der ringförmige Sockel 6, welcher in diesem Ausführungsbeispiel die Durchführungen 11 und 12 aufweist, mittels derer die LEDs auf der Oberseite angesteuert werden.

Der Sockel 6 ist in die topfförmige Metallkappe 2 eingelassen.

Zu erkennen ist ferner der mittige Kanal 5, in welchen ein elektrisches, optisches oder mechanisches Bauelement angeordnet sein kann.

Die Zuleitungen 4 werden um den Kanal 5 auf der Unterseite herumgeführt.

Es versteht sich, dass eine LED-Leuchte mit mehreren, getrennt voneinander angesteuerten LEDs in der Regel mehr als die hier dargestellten zwei Durchführungen für Plus- und Minuspol umfasst. Die Treiberschaltungen zum Ansteuern der LEDs befinden sich vorzugsweise nämlich nicht im hermetisch abgedichteten Bereich der hier dargestellten Leuchte, sondern sind extern angeordnet.

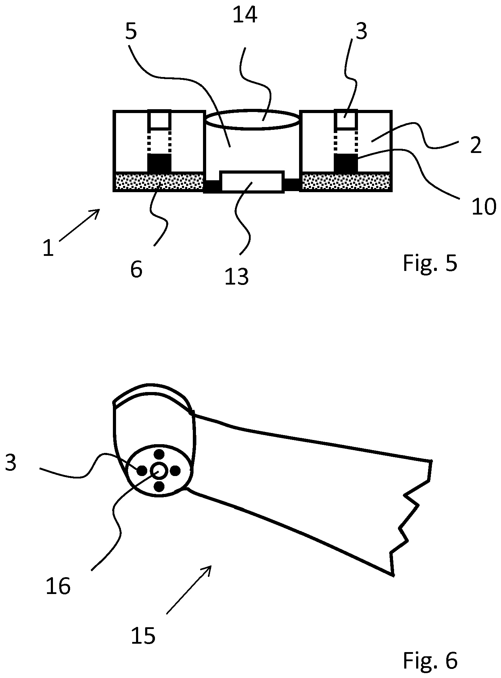

Fig. 5 zeigt schematisch eine hermetisch abgedichtete LED-Leuchte 1. Diese besteht auch in diesem Ausführungsbeispiel aus einem ringförmigen Sockel 6 aus Keramik, auf den eine ebenfalls ringförmige Metallkappe mit einer Mehrzahl von Fenstern 3, durch die das Licht der LEDs 10 austritt, aufgelötet ist.

In dem mittigen Kanal 5 ist ein Bildsensor 13 sowie eine Linse 14 angeordnet.

Fig. 6 zeigt eine schematische Ansicht eines ärztlichen Instruments, und zwar des Kopfstücks eines zahnärztlichen Bohrers.

Zu sehen ist eine Aufnahme 16, in welche die Bohrer eingesetzt werden und sodann mit einer Antriebswelle verbunden sind.

Direkt auf den Kopf des ärztlichen Instruments 15 montiert ist eine erfindungsgemäße, hermetisch abgedichtete LED-Leuchte, deren Licht direkt über die Fenster 3 nach außen abgestrahlt wird.

Unter anderem aufgrund der guten Dichtigkeit des Gehäuses der erfindungsgemäßen Leuchte ist es nicht notwendig, die LED-Leuchte im Gehäuse des ärztlichen Instruments anzuordnen und das abgestrahlte Licht über Lichtleiter nach vorne zu führen.

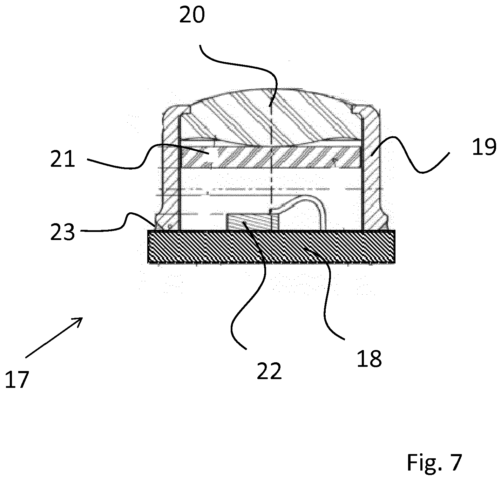

Fig. 7 zeigt eine Ausführungsvariante einer hermetisch abgedichteten LED-Leuchte, welche nicht ringförmig ausgebildet ist, aber mit dem erfindungsgemäßen Verfahren hergestellt wurde.

Die LED-Leuchte 17 umfasst einen Sockel 18 aus einer Keramik, insbesondere aus Aluminiumoxid, auf welchen mittels eines Gold-Zinn-Lots eine Metallkappe 19, insbesondere aus Edelstahl, aufgelötet ist.

Der Sockel 18 ist nicht in die Kappe 19 eingelassen, sondern stirnseitig aufgesetzt.

Die Metallkappe 19 ist randseitig abgeschrägt. In dem dadurch entstehenden Spalt sorgt das dort befindliche Lot 23 für eine bessere Verbindung der Bauteile.

Im Inneren der hermetisch abgedichteten LED-Leuchte 19 ist eine als Chip ausgebildete LED 22 angeordnet, deren Licht zunächst auf einen darüberliegenden Konverter 21 trifft, welcher unterhalb des als Linse ausgebildeten Fensters 20 angeordnet ist.

Auch zum Herstellen dieser LED-Leuchte 17 wird zunächst ein Fenster 20 aus Glas hergestellt, indem dieses in die Metallkappe 19 eingeschmolzen wird. Optional wird die Metallkappe 19 noch mit dem Konverter 21 bestückt.

Sodann wird die Metallkappe 19 mit dem bereits mit der LED 22 bestückten Sockel 18 verlötet.

### Bezugszeichenliste

- 1: LED-Leuchte
- 2: Metallkappe
- 3: Fenster
- 4: Zuleitung
- 5: Kanal
- 6: Sockel
- 7: Kanal
- 8: Einsenkung
- 9: Konverter
- 10: LED
- 11: Durchführung
- 12: Durchführung
- 13: Bildsensor
- 14: Linse
- 15: ärztliches Instrument
- 16: Aufnahme
- 17: LED-Leuchte
- 18: Sockel
- 19: Metallkappe
- 20: Fenster
- 21: Konverter
- 22: LED
- 23: Lot

## Patentansprüche

1. Verfahren zum Herstellen einer hermetisch abgedichteten LED-Leuchte (1), wobei zunächst ein Sockel (6) aus Keramik mit zumindest einer LED (10, 22) bestückt wird und sodann eine Metallkappe (2), mittels eines Metalllots auf den Sockel (6) gelötet wird, wobei die Metallkappe (2) vor dem Verlöten von Metallkappe (2) und Sockel (6) durch Aufschmelzen eines Glases in einem Kanal (7) der Metallkappe (2) mit zumindest einem Glasfenster (3) versehen wird, **dadurch gekennzeichnet, dass** die Metallkappe (2) mittels eines Gold-Zinn-Lots auf den Sockel (6) gelötet wird.

2. Verfahren zum Herstellen einer hermetisch abgedichteten LED-Leuchte (1) nach dem vorstehenden Anspruch, **dadurch gekennzeichnet, dass** die Metallkappe mehrere Glasfenster aufweist und dass die Glasfenster (3) durch Aufschmelzen eines Glases in jeweils einem Kanal (7) der Metallkappe (2) eingebracht werden.

3. Verfahren zum Herstellen einer hermetisch abgedichteten LED-Leuchte (1) nach einem der vorstehenden Ansprüche, **dadurch gekennzeichnet, dass** die Metallkappe (2) beschichtet wird, insbesondere mit einer nickel- und/oder goldhaltigen Beschichtung.

4. Hermetisch abgedichtete LED-Leuchte (1), umfassend einen Sockel (6) aus Keramik , der mit zumindest einer LED (10, 22) bestückt ist, sowie eine Metallkappe (2), die mittels eines Metalllots auf den Sockel (6) gelötet ist, wobei die Metallkappe (2) mit zumindest einen Glasfenster versehen ist, dass in einem Kanal der Metallkappe eingeschmolzen ist, **dadurch gekennzeichnet, dass** die Metallkappe (2) mittels eines Gold-Zinn-Lots auf den Sockel (6) gelötet ist.

5. Hermetisch abgedichtete LED-Leuchte (1) nach Anspruch 4, wobei der Sockel (6) eine Mehrzahl von LEDs (10) umfasst, und wobei die eine Metallkappe (2) eine Mehrzahl von Glasfenstern (3) umfasst, und wobei die LED-Leuchte (1) einen Kanal (5) zum Einbringen eines elektrischen, optischen oder mechanischen Bauelements aufweist, welcher sich durch Sockel (6) und Metallkappe (2) erstreckt.

6. Hermetisch abgedichtete LED-Leuchte (1) nach dem vorstehenden Anspruch 5, **dadurch gekennzeichnet, dass** die LED-Leuchte (1) ringförmig mit mittig angeordnetem Kanal (5) ausgebildet ist.

7. Hermetisch abgedichtete LED-Leuchte (1) nach dem vorstehenden Anspruch 6, **dadurch gekennzeichnet, dass** die Glasfenster (3) ringförmig verteilt sind.

8. Hermetisch abgedichtete LED-Leuchte (1) nach dem vorstehenden Anspruch 7, **dadurch gekennzeichnet, dass** die Glasfenster (3) als Linsen ausgebildet sind.

9. Hermetisch abgedichtete LED-Leuchte (1) nach einem der vorstehenden Ansprüche 5 bis 8, **dadurch gekennzeichnet, dass** die Metallkappe (2) angrenzend zu den Glasfenstern (3) eine Einsenkung (8) umfasst.

10. Hermetisch abgedichtete LED-Leuchte (1) nach einem der vorstehenden Ansprüche 5 bis 9, **dadurch gekennzeichnet, dass** in die Kanäle (7) unterhalb des Glasfensters ein Konverter eingelassen ist.

11. Hermetisch abgedichtete LED-Leuchte (1) nach einem der vorstehenden Ansprüche 5 bis 10, wobei die Leuchte getrennt ansteuerbare LEDs (10) verschiedener Lichtfarbe umfasst, wobei der Abstrahlwinkel des Lichts von zumindest zwei getrennt ansteuerbaren LEDs unterschiedlich ist.

12. Hermetisch abgedichtete LED-Leuchte (1) nach einem der vorstehenden Ansprüche 5 bis 11, **dadurch gekennzeichnet, dass** die Glasfenster (3) als Linsen ausgebildet sind, welche eine unterschiedliche Brennweite und/oder einen unterschiedlichen Öffnungswinkel aufweisen.

13. Ärztliches Instrument (15), umfassend eine hermetisch abgedichtete LED-Leuchte (1) nach einem der Ansprüche 4 bis 12, wobei die LED-Leuchte (1) derart in das ärztliche Instrument (15) eingelassen ist, dass LED-Leuchte direkt nach außen abstrahlt.

## Claims

1. A method for manufacturing a hermetically sealed LED light (1), wherein first, a base (6) made of ceramics is equipped with at least one LED (10, 22), and then a metal cap (2) is soldered to said base (6) using a metallic solder; wherein prior to the soldering of the metal cap (2) to the base (6), at least one glass window (3) is provided in the metal cap (2) by melting a glass in a passage (7) of the metal cap (2);
**characterised in that**
the metal cap (2) is soldered to the base (6) using a gold-tin solder.

2. The method for manufacturing a hermetically sealed LED light (1) according to the preceding claim,
**characterised in that** the metal cap has a plurality of glass windows, and that the glass windows (3) are integrated by melting a glass in a respective passage (7) of the metal cap (2).

3. The method for manufacturing a hermetically sealed LED light (1) according to any one of the preceding claims, **characterised in that** the metal cap (2) is coated, in particular with a coating that contains nickel and/or gold.

4. A hermetically sealed LED light (1), comprising a base (6) made of ceramics, which is equipped with at least one LED (10, 22), and a metal cap (2) which is soldered to the base (6) using a metallic solder; wherein at least one glass window is provided in the metal cap (2), which is fused into a passage of the metal cap;
**characterised in that**
the metal cap (2) is soldered to the base (6) using a gold-tin solder.

5. The hermetically sealed LED light (1) according to claim 4, wherein the base (6) comprises a plurality of LEDs (10); and wherein
the single metal cap (2) has a plurality of glass windows (3), and wherein the LED light (1) has a passage (5) for introducing an electrical, optical, or mechanical component, which passage extends through the base (6) and through the metal cap (2).

6. The hermetically sealed LED light (1) according to the preceding claim 5, **characterised in that** the LED light (1) has an annular shape with centrally arranged passage (5).

7. The hermetically sealed LED light (1) according to the preceding claim 6, **characterised in that** the glass windows (3) are distributed annularly.

8. The hermetically sealed LED light (1) according to the preceding claim 7, **characterised in that** the glass windows (3) have the form of lenses.

9. The hermetically sealed LED light (1) according to any one of the preceding claims 5 to 8,
**characterised in that** the metal cap (2) has a depression adjacent to the glass windows (3).

10. The hermetically sealed LED light (1) according to any one of the preceding claims 5 to 9,
**characterised in that** a converter is disposed in the passages (7) below the glass window.

11. The hermetically sealed LED light (1) according to any one of the preceding claims 5 to 10,
wherein the light comprises separately controllable LEDs (10) of different light colours, wherein at least two separately controllable LEDs have a different light emission angle.

12. The hermetically sealed LED light (1) according to any one of the preceding claims 5 to 11,
**characterised in that** the glass windows (3) are provided in the form of lenses which have a different focal length and/or a different aperture angle.

13. A medical instrument (15), comprising a hermetically sealed LED light (1) according to any one of the preceding claims 4 to 12,
wherein the LED light (1) is embedded in the medical instrument (15) in such a manner that the LED light emits light directly to the outside.

## Revendications

1. Procédé de fabrication d'un appareil d'éclairage à DEL (1) fermé hermétiquement, dans lequel un culot (6) en céramique est dans un premier temps équipé d'au moins une DEL (10, 22) puis un capuchon métallique (2) est brasé sur le culot (6) au moyen d'un métal d'apport de brasage, dans lequel le capuchon métallique (2), avant le brasage du capuchon métallique (2) et du culot (6), est pourvu d'au moins une fenêtre en verre (3) en faisant fondre un verre dans un canal (7) du capuchon métallique (2),
**caractérisé en ce que** le capuchon métallique (2) est brasé sur le culot (6) au moyen d'un apport de brasage d'or et d'étain.

2. Procédé de fabrication d'un appareil d'éclairage à DEL (1) fermé hermétiquement selon la revendication précédente, **caractérisé en ce que** le capuchon métallique comprend plusieurs fenêtres en verre et **en ce que** les fenêtres en verre (3) sont ménagées par fusion d'un verre dans respectivement un canal (7) du capuchon métallique (2) .

3. Procédé de fabrication d'un appareil d'éclairage à DEL (1) fermé hermétiquement selon l'une quelconque des revendications précédentes, **caractérisé en ce que** le capuchon métallique (2) est revêtu, en particulier d'un revêtement contenant du nickel et/ou de l'or.

4. Procédé de fabrication d'un appareil d'éclairage à DEL (1) fermé hermétiquement comportant un culot (6) en céramique qui est équipé d'au moins une DEL (10, 22), ainsi qu'un capuchon métallique (2), qui est brasé sur le culot (6) au moyen d'un métal d'apport de brasage, dans lequel le capuchon métallique (2) est pourvu d'au moins une fenêtre en verre, qui est ménagée par fusion dans un canal du capuchon métallique,
**caractérisé en ce que** :
le capuchon métallique (2) est brasé sur le culot (6) au moyen d'un apport de brasage d'or et d'étain.

5. Appareil d'éclairage à DEL (1) fermé hermétiquement selon la revendication 4, dans lequel le culot (6) comporte une pluralité de DEL (10), et dans lequel l'un des capuchons métalliques (2) comporte une pluralité de fenêtres en verre (3), et l'appareil d'éclairage à DEL (1) comprenant un canal (5) pour l'introduction d'un composant électrique, optique ou mécanique, lequel s'étend à travers le culot (6) et le capuchon métallique (2).

6. Appareil d'éclairage à DEL (1) fermé hermétiquement selon la revendication précédente 5, l'appareil d'éclairage à DEL (1) étant **caractérisé en ce qu'**il présente une forme annulaire au centre de laquelle est agencé le canal (5).

7. Appareil d'éclairage à DEL (1) fermé hermétiquement selon la revendication précédente 6, **caractérisé en ce que** les fenêtres en verre (3) sont réparties de manière annulaire.

8. Appareil d'éclairage à DEL (1) fermé hermétiquement selon la revendication précédente 7, **caractérisé en ce que** les fenêtres en verre (3) sont réalisées sous la forme de lentilles.

9. Appareil d'éclairage à DEL (1) fermé hermétiquement selon l'une des revendications précédentes 5 à 8, **caractérisé en ce que** le capuchon métallique (2) comporte un renfoncement (8) situé dans le prolongement des fenêtres en verre (3).

10. Appareil d'éclairage à DEL (1) fermé hermétiquement selon l'une des revendications précédentes 5 à 9, **caractérisé en ce qu'**un convertisseur est encastré dans les canaux (7) au-dessous de la fenêtre en verre.

11. Appareil d'éclairage à DEL (1) fermé hermétiquement selon l'une des revendications précédentes 5 à 10, l'appareil d'éclairage comportant des DEL (10) à lumière de différente couleur pouvant être commandées séparément, dans lequel l'angle de rayonnement de la lumière d'au moins deux DEL pouvant être commandées séparément est différent.

12. Appareil d'éclairage à DEL (1) fermé hermétiquement selon l'une des revendications précédentes 5 à 11, **caractérisé en ce que** les fenêtres en verre (3) sont réalisées sous la forme de lentilles, lesquelles présentent une distance focale différente et/ou un angle d'ouverture différent.

13. Instrument médical (15), comprenant un appareil d'éclairage à DEL (1) fermé hermétiquement selon l'une des revendications 4 à 12, l'appareil d'éclairage à DEL (1) étant encastré dans l'instrument médical (15) de telle manière que l'appareil d'éclairage à DEL émet une lumière directement vers l'extérieur.
